(19) Europäisches Patentamt
European Patent Office
Office européen des brevets

(11) Veröffentlichungsnummer: **0 189 159 B1**

# (12) EUROPÄISCHE PATENTSCHRIFT

(45) Veröffentlichungstag der Patentschrift: **01.07.92**

(51) Int. Cl.5: **C07D 493/22**, C07H 17/08, A61K 31/70, A61K 31/55, A01N 43/90, //(C07D493/22, 313:00,311:00,311:00,307:00)

(21) Anmeldenummer: **86100674.0**

(22) Anmeldetag: **20.01.86**

Die Akte enthält technische Angaben, die nach dem Eingang der Anmeldung eingereicht wurden und die nicht in dieser Patentschrift enthalten sind.

## (54) 13Beta-Alkyl-milbemycinderivate zur Bekämpfung von Parasiten an Tieren und Pflanzen.

(30) Priorität: **22.01.85 CH 278/85**

(43) Veröffentlichungstag der Anmeldung: **30.07.86 Patentblatt 86/31**

(45) Bekanntmachung des Hinweises auf die Patenterteilung: **01.07.92 Patentblatt 92/27**

(84) Benannte Vertragsstaaten: **DE FR**

(56) Entgegenhaltungen:
**EP-A- 0 144 285**
**US-A- 4 093 629**

(73) Patentinhaber: **CIBA-GEIGY AG**
**Klybeckstrasse 141**
**CH-4002 Basel(CH)**

(72) Erfinder: **Frei, Bruno, Dr.**
**Bündtenstrasse 16**
**CH-4410 Liestal(CH)**
Erfinder: **O'Sullivan, Anthony Cornelius, Dr.**
**Habsburgerstrasse 38**
**CH-4055 Basel(CH)**
Erfinder: **Gehret, Jean-Claude, Dr.**
**Im Aeschfeld 12**
**CH-4147 Aesch(CH)**

(74) Vertreter: **Zumstein, Fritz, Dr. et al**
**Bräuhausstrasse 4**
**W-8000 München 2(DE)**

EP 0 189 159 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 13$\beta$-Alkyl-Milbemycinderivate der nachstehenden Formel I, deren Herstellung sowie deren Verwendung zur Bekämpfung von Schädlingen wie Ekto- und Endoparasiten an Tieren und Pflanzenparasiten.

Bei den erfindungsgemässen Verbindungen handelt es sich um 13$\beta$-Alkyl-Milbemycine der allgemeinen Formel I

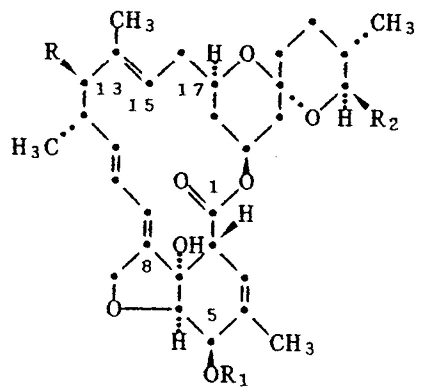

(I)

worin R für $C_1$-$C_{10}$-Alkyl steht; $R_1$ Wasserstoff, eine Silylgruppe oder den Zuckerrest

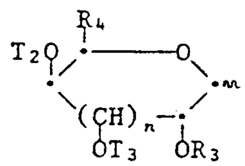

unter Einschluss seiner Stellungsisomeren repräsentiert, wobei n für die Zahl 0 oder 1 steht; $R_4$ Wasserstoff, Methyl oder -$CH_2$-O-$T_1$ bedeutet und $R_4$, $T_1$, $T_2$ und $T_3$ unabhängig voneinander Wasserstoff, Methyl, Benzyl, eine unsubstituierte oder halogensubstituierte $C_1$-$C_6$-aliphatische Acylgruppe, eine Benzoylgruppe, oder eine $C_1$-$C_6$-Alkoxycarbonylgruppe bedeuten, oder wobei $T_1$ und $T_2$ zusammen mit dem Carbonyl-Kohlenstoffatom eines aliphatischen oder aromatischen Aldehyds oder Ketons mit maximal 13 Kohlenstoffatomen ein cyclisches Acetal bilden, $R_3$ für Methyl, Benzyl, Benzoyl, unsubstituiertes oder durch Fluor substituiertes Propionyl, Acetyl, Methoxycarbonyl oder Ethoxicarbonyl steht und $R_2$ für Methyl, Ethyl, Isopropyl oder sek.-Butyl steht.

Formel I repräsentiert somit Milbemycin-abkömmlinge, die eine 13$\beta$-Alkylgruppe enthalten und in 5-Position entweder eine freie OH-Gruppe, eine Silylgruppe oder einen Zuckerrest, insbesondere ein Mono-, Di- oder Trisaccharid aufweisen, das neben der Verknüpfungsstelle zum Makrolid eine OH-Gruppe trägt, die ihrerseits vorzugsweise derivatisiert ist.

Unter dem Begriff Alkyl selbst oder als Bestandteil eines anderen Substituenten sind je nach Zahl der angegebenen Kohlenstoffatome beispielsweise folgenden Gruppen zu verstehen: Methyl, Ethyl, Propyl, Butyl, Pentyl, Hexyl, Heptyl, Octyl, Nonyl, Decyl, usw. sowie die Isomeren, wie z.B. Isopropyl, Isobutyl, tert.-Butyl, Isopentyl usw..

Als geeignete Silylgruppen für $R_1$ kommt der Rest -Si($R_5$)($R_6$)($R_7$) in Frage, wobei $R_5$, $R_6$ und $R_7$ vorzugsweise unabhängig voneinander für $C_1$-$C_4$-Alkyl, Benzyl oder Phenyl stehen und beispielsweise eine der Gruppen Trimethylsilyl, tris(tert.Butyl)silyl, Diphenyl-tert.butyl-silyl, bis(Isopropyl)methylsilyl, Triphenylsilyl usw. und insbesondere tert.Butyl-dimethylsilyl bildet. Die 5-OH-Gruppe kann auch als Benzylether oder Methoxiethoximethylether vorliegen.

In den vorangegangenen Definitionen bedeutet Halogen vor allem Fluor, Chlor oder Brom.

Zur Bildung eines cyclischen Acetals an einem Zuckermolekül eignen sich einfache Aldehyde wie Acetaldehyd, Propionaldehyd, Butyraldehyd, Benzaldehyd oder Ketone wie Acetophenon, Cyclopentanon, Cyclohexanon, Cycloheptanon, Fluorenon, Methylethylketon, vor allem aber Aceton unter Bildung entsprechender Acetonide.

Verbindungen, worin $R_2$ sek.Butyl darstellt, sollen hier und im folgenden gleichfalls zu den Milbemycin-Derivaten gerechnet werden, obwohl sie nach der üblichen Systematik nicht darunter fallen, sondern gemäss US-PS 4.173.571 von Avermectin-Derivaten abgeleitet sind.

Verbindungen der Formel I, worin $R_1$ eine Silylgruppe oder einen Zuckerrest darstellt, lassen sich durch einfache, z.B. hydrolytische Abspaltung dieser Funktionen in die hochaktiven freien 5-Hydroxiderivate ($R_1 = H$) überführen und haben somit Zwischenprodukte-Charakter. Im übrigen wird der biologische Wert dieser Verbindungen durch die Schutzgruppe im Prinzip nicht gemindert.

Die Substituenten R in 13-Position bedeuten in natürlich vorkommenden Milbemycinen ($R_1$ = H; $R_2$ = $CH_3$, $C_2H_5$ oder iso$C_3H_7$) stets Wasserstoff. Bei Avermectinen dagegen steht in der 13-Position ein $\alpha$-L-Oleandrosyl-$\alpha$-L-oleandrose-Rest, der über Sauerstoff in $\alpha$-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-aglykonen, die eine allylische 13$\alpha$-Hydroxy-Gruppe besitzen. Bei den Avermectin-derivaten der vorliegenden Anmeldung liegt die $\Delta^{22,23}$-Doppelbindung stets in hydrierter Form vor.

Folgende Untergruppen von Verbindungen der Formel I sind auf Grund ihrer ausgeprägten parasitiziden und insektiziden Wirkung besonders bevorzugt:

Gruppe Ia: Verbindungen der Formel I, worin R für $C_1$-$C_{10}$-Alkyl steht; $R_1$ die Gruppe -Si($R_5$)($R_6$)($R_7$) repräsentiert, wobei $R_5$, $R_6$ und $R_7$ unabhängig voneinander für $C_1$-$C_4$-Alkyl, Benzyl oder Phenyl stehen; und $R_2$ für Methyl, Ethyl, Isopropyl oder sek.-Butyl steht.

Gruppe Ib: Diejenigen Verbindungen innerhalb der Untergruppe Ia, worin R für $C_1$-$C_4$-Alkyl steht; $R_1$ Trimethylsilyl, tris(tert.-Butyl)-silyl, Diphenyl-tert.-butylsilyl, bis(Isopropyl)methylsilyl, Triphenylsilyl oder tert.-Butyl-dimethylsilyl bedeutet; und $R_2$ für Methyl, Ethyl, Isopropyl oder sek.-Butyl steht.

Gruppe Ic: Verbindungen der Formel I, worin R für $C_1$-$C_{10}$-Alkyl steht; $R_1$ den Zuckerrest

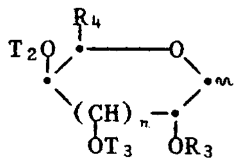

unter Einschluss seiner Stellungsisomeren repräsentiert, wobei n für die Zahl 0 oder 1 steht; $R_4$ Wasserstoff, Methyl oder -$CH_2$-O-$T_1$ bedeutet und $R_4$, $T_1$, $T_2$ und $T_3$ unabhängig voneinander Wasserstoff, Methyl, Benzyl, eine unsubstituierte oder halogensubstituierte $C_1$-$C_6$-aliphatische Acylgruppe, eine Benzoylgruppe, oder eine $C_1$-$C_6$-Alkoxycarbonylgruppe bedeuten, oder wobei $T_1$ und $T_2$ zusammen mit dem Carbonyl-Kohlenstoffatom eines aliphatischen oder aromatischen Aldehyds oder Ketons mit maximal 13 Kohlenstoffatomen ein cyclisches Acetal bilden.

Gruppe Id: Diejenigen Verbindungen im Umfang der Untergruppe der Formel Ic, worin $R_1$ für $C_1$-$C_4$-Alkyl steht; $R_3$ für Methyl, Benzyl, Benzoyl, unsubstituiertes oder durch Fluor substituiertes Propionyl, Acetyl, Methoxycarbonyl oder Ethoxicarbonyl bedeutet; und $R_2$, $R_4$, $T_2$ und $T_3$ die unter Formel Ic angegebenen Bedeutungen haben.

Gruppe Ie: Verbindungen der Formel I, worin R für $C_1$-$C_{10}$-Alkyl steht; $R_1$ Wasserstoff bedeutet; und $R_2$ für Methyl, Ethyl, Isopropyl oder sek.-Butyl steht sind besonders bevorzugt.

Gruppe If: Verbindungen der Formel I, worin R für $C_1$-$C_6$-Alkyl steht; $R_1$ Wasserstoff bedeutet; und $R_2$ für Methyl, Ethyl, Isopropyl oder sek.-Butyl steht.

Gruppe Ig: Verbindungen der Formel I, worin R für $C_1$-$C_4$-Alkyl steht; $R_1$ Wasserstoff bedeutet; und $R_2$ für Methyl, Ethyl, Isopropyl oder sek.-Butyl steht.

Gruppe Ih: Verbindungen der Formel I, worin R für Methyl, Ethyl, n-Propyl oder Isopropyl steht; $R_1$ Wasserstoff bedeutet und $R_2$ für Methyl, Ethyl oder Isopropyl steht.

Besonders bevorzugte Einzelsubstanzen der Formel I sind z.B.:

13$\beta$-n-Hexyl-milbemycin D,
13$\beta$-Methyl-milbemycin D,
13$\beta$-Ethyl-milbemycin D,
13$\beta$-n-Propyl-milbemycin $A_4$,
13$\beta$-iso-Propyl-milbemycin $A_4$,
13$\beta$-Methyl-milbemycin $A_3$

13β-Ethyl-milbemycin $A_3$
13β-Methyl-milbemycin $A_4$
13β-Ethyl-milbemycin $A_4$
13β-iso-Butyl-milbemycin $A_4$
13β-n-Butyl-milbemycin $A_4$.

Pestizid aktive Milbemycine bzw. Milbemycin-Abkömmlinge mit Wasserstoff oder polaren, reaktiven Gruppen, wie Hydroxy, Brom oder Acetoxy in 13-Position sind in den Druckschriften US-A-4,093,629, EP-A-144 285 und US-A-4,173,571 beschrieben.

Die vorliegende Erfindung betrifft nicht nur die Verbindungen der Formel I, sondern gleichermassen das neue Verfahren zu deren Herstellung. Es wurde nämlich gefunden, dass man durch Reaktion mit Trialkylaluminium-Verbindungen der Formel $Al(R)_3$ die nachstehend definierten Allylester der Formel II, worin die allylische $OR_8$-Gruppe sich in der 15-Position des Moleküls befindet, in die Verbindungen der Formel I überführen kann, so dass der einzuführende Substituent R stereospezifisch die 13β-Position des Moleküls einnimmt und nur in untergeordnetem Masse Nebenprodukte liefert, die in 15-Position substituiert sind. Als $R_8$ kommen Acyl-Gruppen in Frage, so z.B.: Formyl, Acetyl, Benzoyl, Ethoxycarbonyl oder $P(=O)(OAlkyl)_2$ wie $P(=O)(OEt)_2$, Alkylsulfonyl-Reste, vorzugsweise Niederalkylsulfonyl, insbesondere Mesyl und in gewissen Fällen auch Tetrahydropyranyl.

Ferner wurde gefunden, dass sich auch aus Verbindungen der Formel II, die eine 13β-$OR_8$-Gruppe enthalten, sich unter Beibehaltung der 13β-Orientierung die Verbindungen der Formel I gewinnen lassen. Das erfindungsgemässe Verfahren bildet somit die Möglichkeit, in der 13β-Position von Milbemycin- oder 13-Deoxi-22,23-dihydro-Avermectinaglykon-Derivaten eine Alkyl-Gruppe R gezielt einzuführen und damit zu hochwirksamen neuen Parasitiziden und Insektiziden der Formel I zu gelangen, die gleichzeitig auch für weitere Derivatisierungen verwendet werden können.

Ein weiterer Gegenstand der vorliegenden Erfindung ist somit ein Verfahren zur Herstellung der Verbindungen der Formel I, das darin besteht, dass man einen Allylester der Formel II

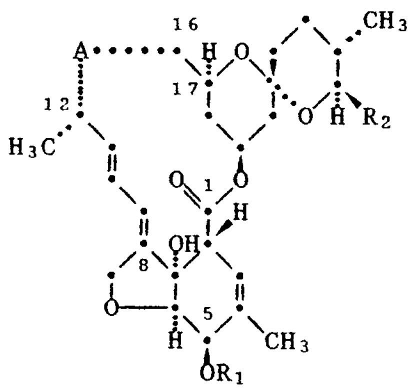

(II)

worin A für eine der Gruppen a oder b

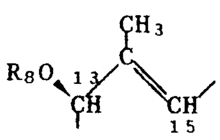 (a) oder 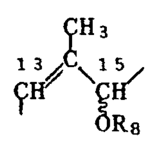 (b)

[= 13β-Ester-$\Delta^{14,15}$] [= $\Delta^{13,14}$-15-Ester]

steht, $R_8$ eine Acylgruppe repräsentiert, $R_1$ Wasserstoff oder vorzugsweise eine Silylgruppe bedeutet und $R_2$ die unter Formel I angegebene Bedeutung hat, mit einer Trialkylaluminium-Verbindung der Formel III

$Al(R)_3$ (III)

behandelt, wobei R die unter Formel I angegebenen Bedeutungen hat, woraufhin man die $R_1$-Silylgruppe,

sofern freie 5-Hydroxi-Verbindungen erwünscht sind, hydrolytisch abspaltet und zur Einführung des Zuckerrestes $R_1$ eine 5-Hydroxi-Verbindung der Formel I mit einem zur Einführung dieser Gruppe geeigneten Zuckerderivat umsetzt.

Das Verfahren wird im allgemeinen in einem reaktions-inerten Lösungsmittel durchgeführt. Geeignete Lösungsmittel sind z.B.: Ether und etherartige Verbindungen wie Dialkylether (Diethylether, Diisopropylether, tert.-Butylmethylether, Dimethoxyethan, Dioxan, Tetrahydrofuran, Anisol, usw.); halogenierte Kohlenwasserstoffe wie Chlorbenzol, Methylenchlorid, Ethylenchlorid, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. Es kann von Vorteil sein, wenn die Reaktion oder Teilschritte davon unter Schutzgasatmosphäre (z.B. Argon, Helium, Stickstoff, etc.) und/oder in absoluten Lösungsmitteln durchgeführt werden. Gewünschtenfalls können Zwischenprodukte aus dem Reaktionsmedium isoliert und falls erwünscht, vor der Weiterreaktion, auf übliche Art und Weise gereinigt werden, z.B. durch Waschen, Digerieren, Extraktion, Umkristallisation, Chromatographie usw. Mann kann jedoch auch auf derartige Reinigungsschritte verzichten und diese erst mit entsprechenden Endprodukten durchführen.

Zur Einführung der 13β-Alkylgruppe geeigneten Trialkylaluminium-Verbindungen sind $(C_1\text{-}C_{10}\text{-Alkyl})_3$Aluminium-Verbindungen, wie z.B. Trimethylaluminium, Triethylaluminium, Triisobutylaluminium, Trihexylaluminium usw.. Die Reaktion wird im allgemeinen im Temperaturbereich -100 °C bis 100 °C, bevorzugt bei -20 °C bis +60 °C durchgeführt. Die Trialkyl-Aluminiumverbindung der Formel III wird dabei in Substanz oder in einem reaktionsinerten Lösungsmittel wie z.B. Hexan, Toluol oder Benzol in mindestens equimolarer Menge zur Lösung der Verbindung der Formel II gegeben.

Nach erfolgter Reaktion wird die Silyl-Schutzgruppe zweckmässigerweise durch Behandlung der Verbindungen der Formel I mit einer verdünnten Säure wie z.B. mit 1 proz. p-Toluolsulfonsäure in Methanol oder mit einer wässrigen HF-Lösung in Acetonitril im Temperaturbereich -20 °C bis 50 °C, bevorzugt bei 0 °C bis 30 °C oder mit Pyridiniumfluorid in Pyridin wieder abgespalten.

Die Herstellung von Verbindungen der Formel I, die an das Sauerstoffatom in der 5-Position einen Kohlenhydratrest gebunden haben, stellt eine Derivatisierung der sehr reaktionssfähigen 5-Hydroxigruppe 13β-Alkyl-Milbemycin mit einem geeigneten Kohlenhydratmolekül dar und wird nach einem der in der Zuckerchemie verwendeten Verknüpfungsmethoden, z.B. nach der Koenigs-Knorr-Synthese, dem Ag-Triflat-Prozess, nach dem sogenannten Orthoester-Verfahren, der Phenylthio-Synthese oder der 2-Pyridylthio-Synthese durchgeführt.

Die als Ausgangssubstanzen eingesetzten Ester der Formel II können aus den zugrundeliegenden Allylalkoholen der Formel IV

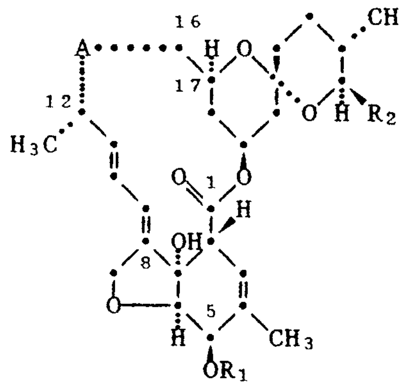

(IV)

worin A für eine der Gruppen a oder b

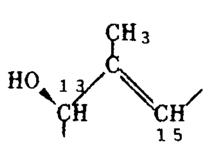 (a) oder 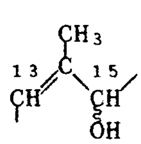 (b)

[= 13β-Hydroxi-$\Delta^{14,15}$]    [= $\Delta^{13,14}$-15-Hydroxi]

steht $R_2$ die unter Formel I angegebenen Bedeutungen hat und $R_1$ für Wasserstoff oder eine unter Formel I genannte Silylgruppe steht; durch übliche, literaturbekannte Acylierungsmethoden wie z.B. durch Reaktionen mit Säurechloriden ($R_8COCl$) oder Säureanhydriden ($(R_8CO)_2O$, wobei $R_8$ die unter Formel II angegebenen Bedeutungen hat, in Gegenwart einer Base (Triethylamin, Pyridin, N,N-Dimethylaminopyridin usw.) in einem eingangs genannten inerten Lösungsmittel z.B. Dichlormethan, Chloroform, usw. im Temperaturbereich von -20°C bis 100°C. bevorzugt bei 0°C bis 70°C hergestellt werden.

Die Verbindungen der Formel IVb [= $\Delta^{13,14}$-15-Hydroxi] lassen sich aus 14,15-Epoxi-Milbemycinen der Formel V gewinnen, worin $R_1$ und $R_2$ die für die Formel I genannten Bedeutungen haben,

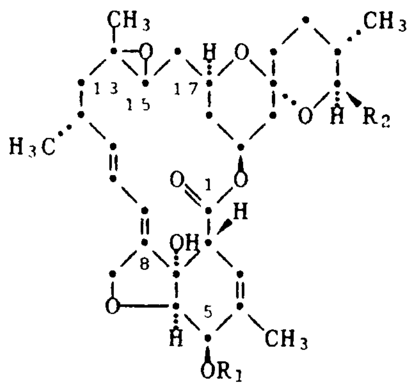

(V)

mit Hilfe des Komplex-Reagenzes $[HN_3]_x/Al(Ethyl)_3]_y$, worin x und y unabhängig voneinander die Zahl 1 oder 2 oder einen Zahlenwert zwischen 1 und 2 darstellen, in inerten trockenen Lösungsmitteln im Temperaturbereich von -20° bis +150°C, vorzugsweise +20° bis +80°C.

Als inerte Lösungsmittel kommen vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan, Anisol in Frage.

Die Reaktion wird vorteilhaft unter Schutzgas, wie Stickstoff oder Argon, durchgeführt.

Stickstoffwasserstoffsäure $HN_3$ lässt sich in statu nascendi in den $[HN_3]_x/[Al(Et)_3]_y$-Komlex überführen, indem man im vorgesehenen trockenen Lösungsmittel oder Lösungsmittel-Gemisch Na-Azid suspendiert und daraus mit einer stärkeren Säure, z.B. $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur Reaktion vorgesehene Epoxi-Verbindung kann gleichfalls bereits vorliegen oder zu einem geeigneten Zeitpunkt zur Lösung dosiert werden.

Die zur Herstellung der Verbindungen IVb verwendeten Ausgangsverbindungen der Formel V lassen sich leicht herstellen durch Epoxidierung der aus der US-PS 3,950,360 bekanntgewordenen und ursprünglich als "Antibiotika B-41-A", später als "Milbemycin-A"-Verbindungen und der aus der US-PS 4,346,171 bekanngewordenen und als "B-41-D" oder "Milbemycin-D" bezeichneten Verbindungen der Formel sowie der aus der US-PS 4,173,571 bekanntgewordenen 13-Deoxi-22,23-dihydro-Avermectine ($R_2$ = sec.Butyl) der nachstehenden Formel VI

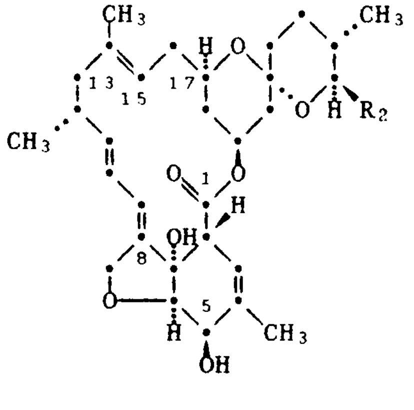

(VI)

$R_2$ = $CH_3$ Milbemycin $A_3$
$R_2$ = $C_2H_5$ Milbemycin $A_4$
$R_2$ = iso$C_3H_7$ Milbemycin D
$R_2$ = sec.$C_4H_9$ 13-Deoxi-22,23-dihydro-C-076-Bla-aglycon.

Die Epoxidierung wird in einer Lösungsmittelphase im Temperaturbereich von -10° bis +20°C, vorzugsweise -5° bis +5°C, durchgeführt.

Die Epoxidierung wird mit Persäuren wie Peressigsäure, Trifluorperessigsäure, Perbenzoesäure, Chlorperbenzoesäure durchgeführt.

Die 13β-Hydroxi-$\Delta^{14,15}$-Verbindungen der Formel IVa lassen sich aus Verbindungen der Formel IIb, worin $R_1$ für eine Schutzgruppe steht, durch Reaktion mit Pyridiniumdichromat [= $(Pyr)_2Cr_2O_7$] herstellen. Man arbeitet hierbei in Dimethylformamid und bei Temperaturen zwischen ca. -10° und +60°C. Die $R_1$-Schutzgruppe wird, sofern gewünscht, anschliessend hydrolytisch abgespalten.

Durch Acylierung oder Silylierung der 5-OH-Gruppe werden alle jene Derivate der Formeln I bis VI hergestellt, bei denen $R_1$ eine andere Bedeutung als Wasserstoff ($R_1$ = OH-Schutzgruppe) hat. Zur Silylierung verwendet man zweckmässigerweise ein Silan der Formel Y-Si($R_5$)($R_6$)($R_7$), worin $R_5$, $R_6$ und $R_7$ einen der eingangs genannten Reste darstellen, und wobei Y eine Silylabgangsgruppe bedeutet. Zu den Silylabgangsgruppen Y zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silylreste $R_1$ in der 5-Position geschieht durch selektive milde Hydrolyse (→ $R_1$ = H) mit z.B. Arylsulfonsäure in alkoholischer Lösung oder nach einer anderen dem Fachmann geläufigen Methode.

Das beschriebene Verfahren zur Herstellung der Verbindungen der Formel I ist in allen Teilschritten ein Bestandteil vorliegender Erfindung.

Ein weiterer Gegenstand vorliegender Erfindung betrifft Schädlingsbekämpfungsmittelgegen Ekto- und Endoparasiten sowie Schadinsekten, die neben üblichen Trägerstoffen und/oder Verteilungsmitteln als mindestens einen Wirkstoff eine Verbindung der Formel I enthalten.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus,

Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon,Dimethylsulfoxid oder Dimethylformamid sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden Können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher

gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Herstellung von Ausgangs- und Zwischenprodukten

#### Beispiel A1: Herstellung von 14,15-Epoxi-milbemycin D (Formel V)

Zu einer Lösung von 550 mg Milbemycin D in 5 ml Dichlormethan wird unter Eiskühlung eine Lösung von 170 mg Chlorperbenzoesäure in 5 ml Dichlormethan gegeben. Nach 1-stündigem Rühren bei 0° bis +5°C werden nochmals 170 mg des Oxidationsmittels hinzugefügt und weitere 30 min gerührt. Nach beendeter Reaktion wird die Lösung in eine eisgekühlte Lösung von Natriumsulfit gegossen und mit Essigsäureethylester extrahiert. Die vereinigten Extrakte werden einmal mit Wasser gewaschen, getrocknet und im Vakuum eingedampft. Das Rohprodukt wird durch Chromatographierung über eine Silicagel-Säule (Elutionsmittel n-Hexan/Essigsäureethylester 20:15) gereinigt. Es werden 450 mg 14,15-Epoxi-milbemycin D als amorphe, weisse Substanz erhalten.

#### Beispiel A2: Herstellung von 15-Hydroxi-$\Delta^{13,14}$-milbemycin D (Formel IVb)

Es werden bei -20°C zu einer Lösung von 2,1 ml (1,75 g, 15,3 mmol) Triethylaluminium in 8,5 ml abs. Diethylether 9,5 ml (0,41 g, 9,53 mmol) einer 6,69 %igen Lösung von $HN_3$ in Diethylether gegeben, die dann bei -10°C unter stark exothermer Reaktion zu 1,8 g (3,15 mmol) 14,15-Epoxi-milbemycin D (in Substanz) gegeben wird. Nach 1 Stunde bei Raumtemperatur werden 4 ml absoluter Ether zugegeben und das gallertartige Reaktionsgemisch wird kräftig gerührt. Nach 4 Stunden wird wie in Vorschrift A1 aufgearbeitet und die Chromatographie an 70 g Kieselgel ($CH_2Cl_2$/Aceton 10:1) ergibt 200 mg (10 %) 14-Azido-15-hydroxi-milbemycin D und 820 g (45 %) 15-Hydroxy-$\Delta^{13,14}$-milbemycin D, Smp.: 151-153°C (aus Methanol).

#### Beispiel A3: Herstellung von 5-O-t-Butyldimethylsilyl-14,15-epoxi-milbemycin D (Formel V)

Eine Lösung von 2,21 g (3,86 mmol) 14,15-Epoxi-milbemycin D, 757 mg (5,02 mmol) t-Butyldimethylchlorsilan und 342 mg (5,02 mmol) Imidazol in 4 ml DMF wird 90 min bei Raumtemperatur gerührt. Anschliessend werden 80 ml Diethylether zugegeben, und das Gemisch wird über 20 g Kieselgel filtriert und eingeengt. Es werden 2,65 g (100 %) 5-O-t-Butyldimethylsilyl-14,15-epoxi-milbemycin D erhalten. $^1$H-NMR (300 MHz. Lösungsmittel $CDCl_3$. Messwerte $\delta$ bezogen auf $Si(CH_3)_4$ = TMS).

0,12 ppm (s) ($(CH_3)_2Si$-O-)
0,92 ppm (s) ((t.-$C_4H_9$)Si-O-)
1,23 ppm (breites s) $C_{14}CH_3$, d.h. Signal der $CH_3$-Gruppe in 14-Position);
2,56 ppm (d; J = 9) ($C_{15}H$, d.h. Signal des Protons in 15-Position).

Aehnlich lässt sich durch Reaktion mit Trimethylsilyl-trifluor-methansulfonat das entsprechende 5-O-Trimethylsilyl-14,15-epoxi-milbemycin D herstellen, Smp. 92-97° C.

Beispiel A4: Herstellung von 5-O-t-Butyldimethylsilyl-15-hydroxi-$\Delta^{13,14}$-milbemycin D (Formel IVb)

Eine Lösung des $HN_3/Et_3Al$-Komplex-Reagenz (hergestellt aus einer Lösung von 4,97 ml Triethylaluminium in 7 ml abs. Tetrahydrofuran (THF) und 9,15 ml einer 2,39 M Lösung von $HN_3$ (21,9 mmol) in abs. Diethylether) wird unter Argon zu einer Lösung von 5,0 g (7,29 mmol) 5-O-Butyldimethylsilyl-14,15-epoxi-milbemycin D in ca. 20 ml abs. THF gegeben, und das Gemisch wird 15 Stunden unter Rückfluss erhitzt. Anschliessend werden bei Raumtemperatur 250 ml Ether, 2 ml Methanol und schliesslich ein Gemisch von 10 g $Na_2SO_4 \cdot 10\ H_2O$ und 10 g Celite zugegeben. Das Gemisch wird filtriert, eingeengt, und die Chromatographie des Rohproduktes an 160 g Kieselgel (O bis 30 % Essigsäureethylester in Hexan) ergibt 2,37 g (47 %) 5-O-t-Butyldimethylsilyl-15-hydroxi-$\Delta^{13,14}$-milbemycin D.
$^1$H-NHR (300 MHz, $CDCl_3$):
1,59 ppm(d; J = 1 Hz), ($C_{14}CH_3$);
4,06 ppm (dd; $J_1$ = 11 Hz; $J_2$ = 4) ($C_{15}H$);
5,15 ppm (d; J = 8 Hz) ($C_{13}H$):
Daneben werden 109 mg (2 %) 13$\beta$-Azido-5-O-t-butyldimethylsilyl-Milbemycin D gewonnen.

Beispiel A5: Herstellung von 14,15-Epoxi-milbemycin $A_4$ ($R_2 = C_2H_5$) [Formel VII]

Zu einer Lösung von 5,7 g (10,5 mmol) Milbemycin $A_4$ in 140 ml Dichlormethan und 120 ml 0,5 M $NaHCO_3$-Lösung wird bei Raumtemperatur eine Lösung von 2,43 g (14,08 mmol) m-Chlorobenzopersäure in 70 ml Dichlormethan tropfenweise zugegeben. Das Gemisch wird 1 Stunde bei Raumtemperatur kräftig gerührt und dann mit 300 ml Dichlormethan verdünnt. Die organische Phase wird mit wässriger $NaHCO_3$-Lösung gewaschen, mit $Na_2SO_4$ getrocknet und eingeengt. Es werden 5,7 g Epoxid als Rohprodukt erhalten.

Beispiel A6: Herstellung von 5-O-t-Butyldimethylsilyl-14,15-epoxi-milbemycin $A_4$ (Formel V)

5,7 g 14,15-Epoxi-milbemycin $A_4$ werden in 10 ml trockenem Dimethylformamid (DMF) gelöst. Bei Raumtemperatur werden 0,63 g (9,16 mmol) Imidazol und 1,4 g (9,34 mmol) t-Butyldimethylchlorsilan zugegeben. Das Gemisch wird 1 Stunde bei Raumtemperatur gerührt und an 150 g Kieselgel chromatographiert (Hexan/Ether 4:1), wobei 2,84 g (40 % d.Th. Ausbeute, bezogen auf Milbemycin $A_4$) des silylierten Epoxi-Derivats erhalten werden.

Beispiel A7: Herstellung von 5-O-t-Butyldimethylsilyl-15-hydroxi-$\Delta^{13,14}$-milbemycin $A_4$ (Formel IVb)

Das Komplex-Reagenz $HN_3$/Al $(Ethyl)_3$ wird wie folgt hergestellt: 2,8 ml (12,2 mmol) $Al(C_2H_5)_3$ in 4 ml abs. THF werden unter Argon bei ca. -20° C langsam mit 5,28 ml (20,4 mmol) einer 10 %igen Lösung von $HN_3$ in abs. Diethylether versetzt. Zu dieser Lösung wird unter Argon eine Lösung von 2,84 g (4,25 mmol) der im Beispiel A6 erhaltenen Verbindung gegeben, und das so erhaltene Gemisch wird 4 Stunden unter Rückfluss erhitzt. Bei Raumtemperatur werden 500 ml Diethylether, 10 g $Na_2SO_4 \cdot 10H_2O$ und 10 g Celite zugegeben, und das Gemisch wird filtriert und eingeengt. Die Chromatographie des Rohproduktes an 100 g Kieselgel (Hexan/Diethylether 7:2) ergibt 1,72 g (60 % d.Th.) der Titel-Verbindung.
$^1$H-NMR (300 MHz, $CDCl_3$; TMS):
1,59 ppm (br. s) ($C_{14}CH_3$);
4,05 ppm (br. s) ($C_{15}H$);
5,15 ppm (d; J = 6 Hz) ($C_{13}H$).

Daneben werden 0,1 g 13$\beta$-Azido-5-O-t-butyldimethylsilyl-milbemycin $A_4$ erhalten.

Beispiel A8: Herstellung von 15-Hydroxi-$\Delta^{13,14}$-milbemycin $A_4$ (Formel IVb)

Die Hydrolyse der im Beispiel A7 genannten Titelverbindung mit einer 1 %igen Lösung von p-Toluolsulfonsäure in Methanol und die Aufarbeitung in Diethylether mit 5 %iger Na-Hydrogencarbonat-Lösung ergibt die Titel-Verbindung.

Beispiel A9: Herstellung von 14,15-Epoxi-milbemycin $A_3$($R_2 = CH_3$) (Formel V)

Nach der Vorschrift des Beispiels A1 werden aus 220 mg Milbemycin $A_3$ in 5 ml Dichlormethan und 75 mg Chlorperbenzoesäure in 5 ml Dichlormethan bei -2° bis +5°C während 1,5 Stunden und Reinigung über eine Silicagel-Säule 190 mg 14,15-Epoxi-Milbemycin $A_3$ gewonnen.

Beispiel A10: Herstellung von 5-O-tert.-Butyldimethylsilyl-14,15-epoxi-milbemycin $A_3$ (Formel V)

Nach der Vorschrift des Beispiels A3 werden aus 190 mg 14,15-Epoxi-Milbemycin $A_3$ und 120 mg tert.-Butyldimethylchlorsilan in Gegenwart von Imidazol 217 mg der Titel-Verbindung erhalten.

Beispiel A11: Herstellung von 5-O-tert.-Butyldimethylsilyl-15-hydroxi-$\Delta^{13,14}$-milbemycin $A_3$ (Formel IVb)

Analog zur Epoxid-Spaltung des Beispiels A7 werden aus 210 mg 5-O-tert.-Butyldimethylsilyl-14,15-epoxi-milbemycin $A_3$ in absolutem Diethylether mit Hilfe des Komplex-Reagenz $HN_3/Et_3Al$ unter Argon nach anschliessender Reinigung 203 mg der Titelverbindung erhalten.
$^1$H-NMR (300 HMz, $CDCl_3$; TMS):
1,58 ppm (br. s) ($C_{14}CH_3$);
4,05 ppm (br. s) ($C_{15}H$);
5,15 ppm (d; J=6 Hz)($C_{13}H$).

Beispiel A12: Herstellung von 15-Hydroxi-$\Delta^{13,14}$-milbemycin $A_3$ (Formel IVb)

Analog zu Beispiel A1 wird das Reagenz $HN_3/Al(C_2H_5)_3$ frisch hergestellt und bei -10°C zu einer Lösung von 830 mg (3,05 mmol) 14,15-Epoxi-milbemycin $A_3$ in 7 ml trockenem Diethylether getropft. Nach der Aufarbeitung werden 385 mg 15-Hydroxi-$\Delta^{13,14}$-milbemycin $A_3$ und 92 mg 14-Azido-15-hydroxi-milbemycin $A_3$ erhalten.

Beispiel A13: Herstellung von 13-Deoxi-14,15-epoxi-22,23-dihydroavermectin-Bla-aglykon ($R_2$ = sec.$C_4H_9$) (Formel V)

Analog zu Beispiel AS erhält man aus 520 mg 13-Deoxi-22,23-dihydro-avermectin-Bla-aglykon [Tetrahedron Letters, Vol. 24, No. 148, pp. 5333-5336 (1983)] und 210 mg m-Chlorbenzopersäure in 20 ml Dichlormethan 510 mg der Titelverbindung.

Beispiel A14: Herstellung von 5-O-t.Butyldimethylsilyl-13-deoxi-14,15-epoxi-22,23-dihydro-avermectin-Bla-aglykon (Formel V)

Analog zu Beispiel A5 erhält man aus 220 mg der Titelverbindung von Beispiel 14 und 55 mg tert.Butyldimethylchlorsilan in Gegenwart von 25 mg Imidazol in 5 ml trockenem DMF 108 mg der Titelverbindung.

Beispiel A15: Herstellung von 13-Deoxi-15-hydroxi-$\Delta^{13,14}$-22,23-dihydro-avermectin-Bla-aglykon (Formel IVb)

Analog zu Beispiel A2 erhält man aus 220 mg der Titelverbindung von Beispiel A14 mit dem Komplex-Reagenz, bestehend aus 320 mg $Al(C_2H_5)_3$ und 110 mg einer 6,96 %igen Lösung von $HN_3$ in total 16 ml trockenem Diethylether 112 mg der Titelverbindung. Daneben werden 61 mg 13-Deoxi-14-azido-15-hydroxi-22,23-dihydro-avermectin-Bla-aglykonerhalten.

Beispiel A16: a) Herstellung von 5-O-tert.Butyldimethylsilyl-13β-hydroxi-milbemycin D und von 13β-Hydroxi-milbemycin D (Formel IVa)

Eine Lösung bestehend aus 286 mg (0,41 mmol) 5-O-tert.Butyldimethylsilyl-15-hydroxi-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Bei der Chromatographie des Rohproduktes an 20 g Kieselgel (Ether/Hexan 1:2) werden 165 mg (57 %) 5-O-t-Butyldimethylsilyl-13β-hydroxi-milbemycin D erhalten.
$^1$H-NMR (300 MHz; $CDCl_3$; TMS):

1,59 ppm (br. s)($C_{14}CH_3$)
3,70 ppm (d; J = 10 Hz)($C_{13}H$).

105 mg (0,153 mmol) der so gewonnenen Verbindung werden mit 1 ml einer 1 %igen Lösung von p-Toluolsulfonsäure in Methanol 1 Stunde bei Raumtemperatur gerührt. Das Gemisch wird mit 20 ml Ether verdünnt, durch Kieselgel filtriert, eingeengt, und der Rückstand wird an ca. 10 g Kieselgel chromatographiert (Aceton/Dichlormethan 1:4), wobei 73 mg (83 %) 13β-Hydroxi-Milbemycin D erhalten werden.
$^1$H-NMR (300 MHz; $CDCl_3$; TMS):
1,58 ppm (br.s)($C_{14}CH_3$)
3,71 ppm (d; J = 10 Hz)($C_{13}H$).

b) Herstellung von 5-O-tert.-Butyldimethylsilyl-13β-hydroxi-milbemycin $A_4$

Setzt man analog zur Vorschrift a) als Ausgangsmaterial 5-O-tert.-Butyldimethylsily-15-hydroxi-$\Delta^{13,14}$-milbemycin $A_4$ ein, so erhält man die Titelverbindung, die folgende physikalische Daten aufweist:
$^1$H-NMR (300 MHz; $CDCl_3$; TMS):
3,05 ppm (t; J = 9 Hz) ($C_{25}H$)
3,71 ppa (dd; J = 3 und 10 Hz) ($C_{13}H$)
Massenspektrum (FD) m/e: 672 ($M^+$; $C_{38}H_{60}O_8Si$).

Beispiel A17: a) Herstellung von 5-O-tert.Butyldimethylsilyl-13β-acetoxi-milbemycin D

Eine Lösung von 200 mg (0,29 mmol) 5-O-tert.Butyldimethylsilyl-13β-hydroxi-milbemycin D und 1 ml Pyridin in 2 ml Essigsäureanhydrid wird 2 Std. bei Raumtemperatur gerührt. Die Aufarbeitung in Diethylether liefert 212 mg 5-O-tert.Butyldimethylsilyl-13β-acetoxi-milbemycin D in Form eines amorphen Pulvers.

b) Herstellung von 5-O-tert.-Butyldimethylsilyl-13β-acetoxi-milbemycin $A_4$

Setzt man analog zur Vorschrift a) als Ausgangsmaterial 5-O-tert.-Butyldimethylsilyl-13β-hydroxi-milbemycin $A_4$ ein, so erhält man die Titelverbindung, die folgende physikalische Daten aufweist:
$^1$H-NMR (360 MHz; $CDCl_3$; TMS):
1,53 ppm (s) ($C_{14}CH_3$)
2,03 ppm (s) ($CH_3COO$)
4,94 ppm (d; J = 10 Hz) ($C_{13}H$)
Massenspektrum (FD) m/e: 714 ($M^+$; $C_{40}H_{62}O_9Si$).

Beispiel A18: a) Herstellung von 5-O-tert.-Butyldimethylsilyl-15-acetoxi-$\Delta^{13,14}$-milbemycin D

Eine Lösung von 627 mg (0,914 mmol) 5-O-tert.-Butyldimethylsilyl-15-hydroxi-$\Delta^{13,14}$-milbemycin D in 2 ml Essigsäureanhydrid und 2 ml Pyridin wird 0,5 Std. bei Raumtemperatur gerührt. Die Aufarbeitung in Diethylether mit 5 proz. wässriger $NaHCO_3$-Lösung und 1 M HCl und Filtration durch Kieselgel ergibt 624 mg (94 %) 5-O-tert.Butyldimethylsilyl-15-acetoxi-$\Delta^{13,14}$-milbemycin D.
$^1$H-NMR (300 MHz; $CDCl_3$; TMS):
1,58 ppm (br. s) ($C_{14}CH_3$)
1,79 ppm (br. s) ($C_4CH_3$)
2,02 ppm (s) ($CH_3COO$)
5,12 - 5,26 ppm (m) ($C_{10}H$; $C_{13}H$; $C_{15}H$)

b) Herstellung von 5-O-tert.-Butyldimethylsilyl-15-acetoxi-$\Delta^{13,14}$-milbemycin $A_4$

Setzt man gemäss Vorschrift a) als Ausgangsprodukt 5-O-tert.-Butyldimethylsilyl-15-hydroxi-$\Delta^{13,14}$-milbemycin $A_4$ ein, so erhält man die Titelverbindung, die folgende physikalische Daten aufweist:
$^1$H-NMR (250 MHz; $CDCl_3$; TMS):
1,59 ppm (s) ($C_{14}CH_3$)
2,03 ppm (s) ($CH_3COO$)
3,02 ppm (t; J = 8 Hz) ($C_{25}H$)
3,88 ppm (d; J = 6 Hz) ($C_6H$)
Massenspektrum m/e: 714 ($M^+$; $C_{40}H_{62}O_9Si$), 639, 579, 497, 472, 437, 413, 412, 394, 349.

c) Herstellung von 5-O-tert.-Butyldimethylsilyl-15-acetoxi-$\Delta^{13,14}$-milbemycin $A_3$

Die Herstellung verläuft völlig analog zu a) und b) jedoch ausgehend von 5-O-tert.-Butyldimethylsilyl-15-hydroxi-$\Delta^{13,14}$-milbemycin $A_3$.

Herstellung von Endprodukten der Formel I

Beispiel H1: Herstellung von 13$\beta$-Methyl-milbemycin D

Zu einer Lösung von 203 mg (0,28 mmol) 5-0-tert.Butyldimethylsilyl-15-acetoxi-$\Delta^{13,14}$-milbemycin D in 2 ml Dichlormethan wurden unter Argon bei 0 °C 1,2 ml einer 17-proz. Lösung von Trimethylaluminium in Toluol unter Rühren zugetropft. Die Lösung wurde 2 Std. bei Raumtemperatur gerührt, dann wurden 0,3 ml Methanol zugetropft, und das Gemisch wurde mit Diethylether verdünnt und mit Celite gerührt. Die Filtration durch Kieselgel (Laufmittel Diethylether) ergab 177 mg 5-O-tert.Butyldimethylsilyl-13$\beta$-methyl-milbemycin D.

Eine Lösung dieses Materials in 0,5 ml Dichlormethan wurde zusammen mit 1 ml einer 40-proz. wässrigen Lösung von HF in Acetonitril (5:95) 1 Std. bei Raumtemperatur gerührt. Das Gemisch wurde in Diethylether aufgearbeitet und durch Kieselgel filtriert. HPLC ($SiO_2$; 0,5 % Methanol in Dichlormethan; Druck 50 bar) des Rohprodukts (154 mg) ergab 116 mg (57 %) 13$\beta$-Methyl-milbemycin D.

$^1$H-NMR (300 MHz; $CDCl_3$; TMS):
1,01 ppm (d, J = 6,7 Hz) ($C_{13}CH_3$)
5,03 ppm (dd; J = 10,5 und 4,6 Hz) ($C_{15}H$)
Massenspektrum m/e: 570 ($M^+$; $C_{34}H_{50}O_7$), 442, 292, 273, 262, 210, 209, 181, 163, 152, 151.

Beispiel H2: a) Herstellung von 13$\beta$-Ethyl-milbemycin D und 15-Ethyl-$\Delta^{13,14}$-milbemycin D

Zu einer Lösung von 340 mg (0,47 mmol) 5-0-tert.Butyldimethylsilyl-15-acetoxi-$\Delta^{13,14}$-milbemycin D in 2 ml Dichlormethan wurden unter Argon bei 0 °C 0,75 ml (0,63 g; 5,5 mmol) Triethylaluminium unter Rühren zugetropft. Die Lösung wurde 1 Std. bei Raumtemperatur gerührt, dann wurde mit Diethylether verdünnt, das Gemisch mit Celite/$Na_2SO_4$:10 $H_2O$ (1:1) versetzt und 1 Std. gerührt. Die Filtration durch Kieselgel (Laufmittel Diethylether) ergab 258 mg eines Gemisches, welches in 0,5 ml Dichlormethan gelöst und zusammen mit 1 ml einer 4 proz. wässrigen Lösung von HF in Acetonitril (5:95) 1 Std. bei Raumtemperatur gerührt wurde. Die Aufarbeitung in Diethylether, Filtration durch Kieselgel (Laufmittel Diethylether) und HPLC (Reversed Phase: Wasser/Methanol 1:9; Druck 50 mbar) des Rohprodukts (183 mg) ergab 88 mg (32 %) 13$\beta$-Ethyl-milbemycin D.

$^1$H-NMR (300 MHz; $CDCl_3$; TMS):
0,73 ppm (t, J = 7,2 Hz) ($C_{13}CH_2CH_3$)
5,03 ppm (dd; J = 10,5 und 4,4 Hz) ($C_{15}H$)
Massenspektrum m/e: 584 ($M^+$; $C_{35}H_{52}O_7$), 456, 287, 276, 210, 209, 181, 163, 151.
und 57 mg (21 %) 15-Ethyl-$\Delta^{13,14}$-milbemycin D.
$^1$H-NMR (300 MHz; $CDCl_3$; TMS):
3,03 ppm (m) ($C_{12}H$)
4,93 ppm (dd; J = 8,7 und 1,2 Hz) ($C_{13}H$)
Massenspektrum m/e: 584 ($M^+$; $C_{35}H_{52}O_7$), 456, 438, 277, 276, 206, 181, 171, 163, 151, 150, 149.

Analog zur Vorschrift H2a werden durch Reaktion mit den entsprechenden Trialkylverbindungen die nachfolgend in H2b bis H2h genannten Milbemycine der Formel I erhalten:

H2b) 13$\beta$-Methyl-milbemycin $A_4$

$^1$H-NMR (250 MHz; $CDCl_3$; TMS):
3,07 ppm (dt; J = 12 und 10 Hz) ($C_{25}H$)
5,05 ppm (dd; j = 10 und 5 Hz) ($C_{15}H$)
Massenspektrum (FD) m/e: 556 ($M^+$; $C_{33}H_{48}O_7$).

H2c) 13$\beta$-Ethyl-milbemycin $A_4$

$^1$H-NMR (250 MHz; $CDCl_3$; TMS):

3,03 ppm (breites t; J = 10 Hz) ($C_{25}H$)
5,02 ppm (dd; J = 10 und 7 Hz) ($C_{15}H$)
Massenspektrum (FD) m/e: 570 ($M^+$; $C_{34}H_{50}O_7$).

H2d) 13β-n-Hexyl-milbemycin D

$^1$H-NMR (250 MHz; $CDCl_3$; TMS):
3,08 ppm (d; J = 8 Hz) ($C_{25}H$)
5,00 ppm (breites t; J = 8 Hz) ($C_{15}H$)
Massenspektrum (FD) m/e: 640 ($M^+$; $C_{39}H_{60}O_7$).

H2e) 13β-n-Butyl-milbemycin $A_4$

$^1$H-NMR (250 MHz; $CDCl_3$; TMS):
3,03 ppm (breites t; J = 10 Hz) ($C_{25}H$)
5,02 ppm (breites t; J = 10 Hz) ($C_{15}H$)
Massenspektrum (FD) m/e: 598 ($M^+$; $C_{36}H_{54}O_7$).

H2f) 13β-Isobutyl-milbemycin $A_4$

$^1$H-NMR (250 MHz; $CDCl_3$; TMS):
3,09 ppm (breites t; J = 10 Hz) ($C_{25}H$)
5,05 ppm (dd; J = 10 und 7 Hz) ($C_{15}H$)
Massenspektrum (FD) m/e: 598 ($M^+$; $C_{36}H_{54}O_7$).

H2g) 13β-Methyl-milbemycin $A_3$

$^1$H-NMR (300 MHz; $CDCl_3$; TMS):
3,27 ppm (m) ($C_{25}H$)
5,06 ppm (dd; J = 10 und 6 Hz) ($C_{15}H$)
Massenspektrum (FD) m/e: 542 ($M^+$; $C_{32}H_{46}O_7$).

H2h) 13β-Ethyl-milbemycin $A_3$

$^1$H-NMR (300 MHz; $CDCl_3$; TMS):
3,25 ppm (m) ($C_{25}H$)
5,06 ppm (dd; J = 10 und 6 Hz) ($C_{15}H$)
Massenspektrum (FD) m/e: 556 ($M^+$; $C_{33}H_{48}O_7$)

Beispiel H3: Herstellung von 13β-Methyl-milbemycin-D

Aus 5-0-tert.Butyldimethylsilyl-13β-acetoxi-milbemycin-D.Zu einer Lösung von 14 mg (0,019 mmol) 5-0-tert.Butyldimethylsilyl-13β-acetoxi-milbemycin D in 0,5 ml Dichlormethan werden unter Argon bei 0°C 0,5 ml einer 17-proz. Lösung von Trimethylaluminium in Toluol unter Rühren zugetropft. Die Lösung wird über Nacht bei 5°C gerührt. Die Aufarbeitung wie unter H1 ergibt 10 mg 5-0-tert.-Butyldimethylsilyl-13β-methyl-milbemycin D.

Eine Lösung dieses Materials in 0,5 ml Dichlormethan wird zusammen mit 1 ml einer 40 proz. wässrigen Lösung von HF in Acetonitril, (5:95) 1 Std. bei Raumtemperatur gerührt. Das Gemisch wird in Diethylether aufgearbeitet und durch Kieselgel filtriert, wobei 8 mg 13β-Methyl-milbemycin D anfallen.

Beispiel H4: Herstellung von 13β-Methyl-5-O-(2,3,4,6-tetra-O-acetyl-1-O-acetyl-1-O-glucopyranosyl)-milbemycin A4

Zu einer Lösung von 49 mg (0,088 mmol) 13-β-Methyl-milbemycin A4, 300 mg (0,72 mmol) 1-Brom-2,3,4,6-tetra-O-acetylglucose und 140 mg (1,1 mmol) Diisopropylethylamin in 30 ml absolutem Diethylaether wird bei Raumtemperatur 185 mg (0,72 mmol) Ag-Triflat gegeben. Man lässt unter Lichtausschluss 15 Stunden rühren, und filtert anschliessend den beige-farbenen Niederschlag ab. Das Filtrat wird mit 100 ml Diethylether verdünnt, zweimal mit je 15 mlIN $NaHCO_3$-Lösung und dann zweimal mit je 15 ml Wasser

gewaschen. Nach dem Trocknen über Natriumsulfat wird die Lösung eingeengt und über Silicagel (Laufmittel Dichlormethan/Diethylether 7:1) gereinigt. Nach der Gefriertrocknung erhält man 74 mg (95 % d. Th.) eines weissen amorphen Pulvers.
$^1$H-NMR (250 MHz; $CDCl_3$; TMS):
3,10 ppm (breites t; J = 10 Hz) ($C_{25}H$)
2,08 ppm (s) (4 $CH_3COO$)
Massenspektrum (Feld-Desorptionsspektrum): m/e 886 ($M^+$ $C_{47}H_{66}O_{16}$)

Beispiel H5: Herstellung von 13β-Methyl-5-O-(2,3,4,6-tetra-O-acetyl-1-O-galactopyranosyl)-milbemycin A4

Zu einer Lösung von 50 mg (0,088 mmol) 13-β-Methyl-milbemycin A4, 300 mg (0,72 mmol) 1-Brom-2,3,4,6-tetra-O-acetylgalactose und 140 mg (1,1 mmol) Diisopropylethylamin in 50 ml absolutem Diethylaether wird bei Raumtemperatur 185 mg (0,72 mmol) Ag-Triflat gegeben. Man lässt unter Lichtausschluss 20 Stunden rühren, und filtert anschliessend den fast farblosen Niederschlag ab. Das Filtrat wird mit 100 ml Diethylether verdünnt, zweimal mit je 15 mlIN $NaHCO_3$-Lösung und dann zweimal mit je 25 ml Wasser gewaschen. Nach dem Trocknen über Magnesiumsulfat wird die Lösung eingeengt und über Silicagel (Laufmittel Dichlormethan/Diethylether 7:1) gereinigt. Nach der Gefriertrocknung erhält man 77 mg (95 % d. Th.) eines weissen amorphen Pulvers.
$^1$H-NMR (300 MHz; $CDCl_3$; TMS):
3,10 ppm (dt; J = 2 und 10 Hz)
2,05 ppm (s) (3 $CH_3COO$)
2,10 ppm (s) (1 $CH_3COO$)
Massenspektrum (Feld-Desorptionsspektrum): m/e 886 ($M^+$; $C_{47}H_{66}O_{16}$)
Analog zu den beschriebenen Arbeitsweisen werden auch die nachfolgend genannten Verbindungen der Formel I hergestellt; wobei die nachfolgenden Tabellen keinen limitierenden Charakter haben:

Tabelle 1: Typische Vertreter von Verbindungen der Formel I, worin $R_1$ für Wasserstoff steht

| Verb. Nr. | $R_2$ | R |
|---|---|---|
| 1.1 | $CH_3$ | $CH_3$ |
| 1.2 | $CH_3$ | $C_2H_5$ |
| 1.3 | $CH_3$ | $C_3H_7$-n |
| 1.4 | $CH_3$ | $C_3H_7$-iso |
| 1.5 | $CH_3$ | $C_4H_9$-n |
| 1.6 | $CH_3$ | $C_4H_9$-sek |
| 1.7 | $CH_3$ | $C_4H_9$-iso |
| 1.8 | $CH_3$ | $C_5H_{11}$-n |
| 1.9 | $CH_3$ | $C_6H_{13}$-n |
| 1.10 | $CH_3$ | $C_7H_{15}$-n |
| 1.11 | $CH_3$ | $C_8H_{17}$-n |
| 1.12 | $CH_3$ | $C_9H_{18}$-n |
| 1.13 | $CH_3$ | $C_{10}H_{21}$-n |
| 1.14 | $C_2H_5$ | $CH_3$ |
| 1.15 | $C_2H_5$ | $C_2H_5$ |
| 1.16 | $C_2H_5$ | $C_3H_7$-iso |
| 1.17 | $C_2H_5$ | $C_4H_9$-n |
| 1.18 | $C_2H_5$ | $C_4H_9$-sek |
| 1.19 | $C_2H_5$ | $C_4H_9$-iso |
| 1.20 | $C_2H_5$ | $C_5H_{11}$-n |
| 1.21 | $C_2H_5$ | $C_6H_{13}$-n |
| 1.22 | $C_3H_7$-iso | $CH_3$ |
| 1.23 | $C_3H_7$-iso | $C_2H_5$ |
| 1.24 | $C_3H_7$-iso | $C_3H_7$-n |
| 1.25 | $C_3H_7$-iso | $C_3H_7$-iso |
| 1.26 | $C_3H_7$-iso | $C_4H_9$-n |
| 1.27 | $C_3H_7$-iso | $C_4H_9$-sek |
| 1.28 | $C_3H_7$-iso | $C_4H_9$-iso |
| 1.29 | $C_4H_9$-sek | $CH_3$ |
| 1.30 | $C_4H_9$-sek | $C_2H_5$ |

Tabelle 1 (Fortsetzung)

| Verb. Nr. | $R_2$ | R |
|---|---|---|
| 1.31 | $C_4H_9$-sek | $C_3H_7$-n |
| 1.32 | $C_4H_9$-sek | $C_3H_7$-iso |
| 1.33 | $C_4H_9$-sek | $C_4H_9$-n |
| 1.34 | $C_4H_9$-sek | $C_4H_9$-sek |
| 1.35 | $C_4H_9$-sek | $C_4H_9$-tert |
| 1.36 | $C_4H_9$-sek | $C_4H_9$-iso |
| 1.37 | $C_3H_7$-iso | $C_6H_{13}$-n |
| 1.38 | $C_3H_7$-iso | $C_5H_{11}$-n |
| 1.39 | $C_2H_5$ | $C_3H_7$-n |

Tabelle 2

| Typische Vertreter von Verbindungen der Formel I, worin $R_1$ eine Silylgruppe darstellt | | | |
|---|---|---|---|
| Verb. Nr. | $R_2$ | R | $R_1$ |
| 2.1 | $CH_3$ | $CH_3$ | tert.-Butyldimethylsilyl |
| 2.2 | $CH_3$ | $C_2H_5$ | tert.-Butyldimethylsilyl |
| 2.3 | $CH_3$ | $C_3H_7$-n | tert.-Butyldimethylsilyl |
| 2.4 | $CH_3$ | $C_3H_7$-iso | tert.-Butyldimethylsilyl |
| 2.5 | $CH_3$ | $C_4H_9$-n | tert.-Butyldimethylsilyl |
| 2.6 | $CH_3$ | $C_4H_9$-sek | tert.-Butyldimethylsilyl |
| 2.7 | $CH_3$ | $C_4H_9$-tert | tert.-Butyldimethylsilyl |
| 2.8 | $C_2H_5$ | $CH_3$ | tert.-Butyldimethylsilyl |
| 2.9 | $C_3H_7$ | $CH_3$ | tert.-Butyldimethylsilyl |
| 2.10 | $C_3H_7$-iso | $CH_3$ | tert.-Butyldimethylsilyl |
| 2.11 | $C_4H_9$-n | $CH_3$ | tert.-Butyldimethylsilyl |
| 2.12 | $C_4H_9$-sek | $CH_3$ | tert.-Butyldimethylsilyl |
| 2.13 | $C_4H_9$-iso | $CH_3$ | tert.-Butyldimethylsilyl |
| 2.14 | $CH_3$ | $CH_3$ | Trimethylsilyl |
| 2.15 | $CH_3$ | $CH_3$ | tris(tert.-Butyl)silyl |
| 2.16 | $CH_3$ | $CH_3$ | Diphenyl-tert.-butylsilyl |
| 2.17 | $CH_3$ | $CH_3$ | bis(Isopropyl)methylsilyl |
| 2.18 | $CH_3$ | $CH_3$ | Triphenylsilyl |

Tabelle 3: Typische Vertreter von Verbindungen der Formel I, worin $R_1$ für die Gruppe

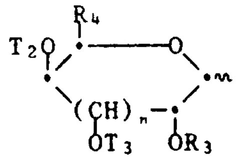

steht und $R_2$ und R Methyl bedeuten sind (In den Fällen, in denen $R_2$ und R andere Bedeutungen haben wird dies speziell vermerkt):

| Verb. Nr. | $R_1$ |
|---|---|
| 3.1 | 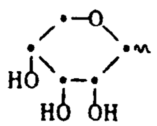 $C_5$-D-ribose |
| 3.2 | 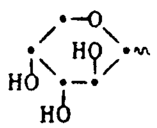 $C_5$-D-arabionose |
| 3.3 | 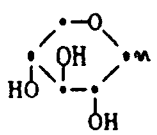 $C_5$-D-xylose |
| 3.4 | 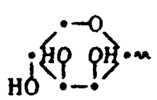 $C_5$-D-lyxose |
| 3.5 | 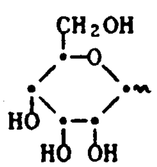 $C_6$-D-allose |

Tabelle 3 (Fortsetzung)

| Verb. Nr. | $R_1$ |
|---|---|
| 3.6 | 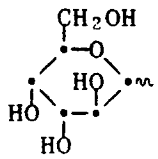<br>$C_6$-D-altrose |
| 3.7 | 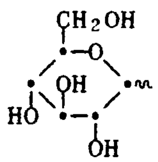<br>$C_6$-D-glukose |
| 3.8 | 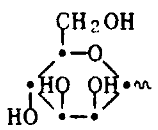<br>$C_6$-D-mannose |
| 3.9 | 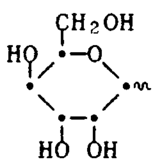<br>$C_6$-D-gulose |
| 3.10 | 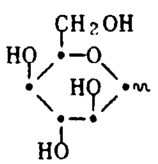<br>$C_6$-D-idose |
| 3.11 | 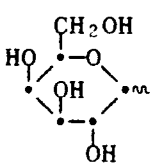<br>$C_6$-D-galactose |

Tabelle 3 (Fortsetzung)

| Verb. Nr. | $R_1$ |
|---|---|
| 3.12 | 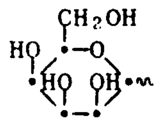<br>$C_6$-D-talose |
| 3.13 | 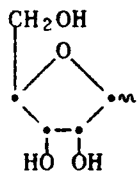<br>$C_5$-D-ribose |
| 3.14 | 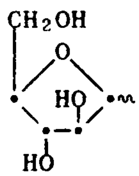<br>$C_5$-D-arabinose |
| 3.15 | 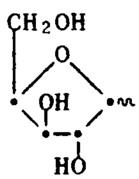<br>$C_5$-D-xylose |
| 3.16 | 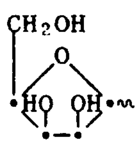<br>$C_5$-D-lyxose |
| 3.17 | 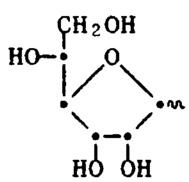<br>$C_6$-D-allose |

Tabelle 3 (Fortsetzung)

| Verb. Nr. | $R_1$ |
|---|---|
| 3.18 | 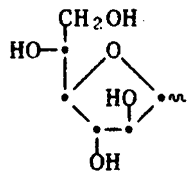 $C_6$-D-altrose |
| 3.19 | 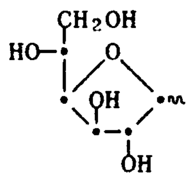 $C_6$-D-glukose |
| 3.20 | 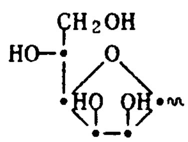 $C_6$-D-mannose |
| 3.21 | 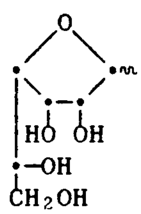 $C_6$-D-gulose |
| 3.22 | 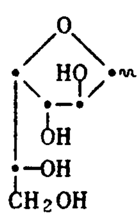 $C_6$-D-idose |

Tabelle 3 (Fortsetzung)

| Verb. Nr. | $R_1$ | $R_2$ |
|---|---|---|
| 3.23 | 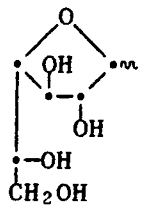 $C_6$-D-galactose | |
| 3.24 | 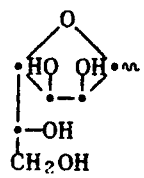 $C_6$-D-talose | |
| 3.25 | 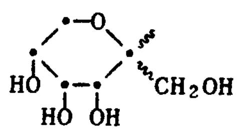 $C_6$-D-psicose | |
| 3.26 | 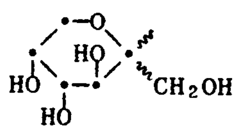 $C_6$-D-fructose | |
| 3.27 | 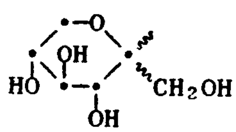 $C_6$-D-sorbose | |

Tabelle 3 (Fortsetzung)

| Verb. Nr. | $R_1$ | $R_2$ |
|---|---|---|
| 3.28 | 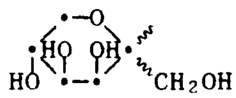<br>$C_6$-D-tagatose | |
| 3.29 | 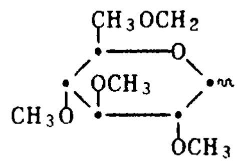<br>2,3,4,6-Tetra-O-methyl-($C_6$-D-glucose) | |
| 3.30 | 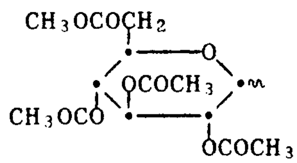<br>2,3,4,6-Tetra-O-acetyl-($C_6$-D-glucose) | $C_2H_5$ |
| 3.31 | 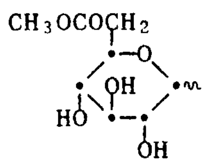<br>6-O-Acetyl-($C_6$-D-glucose) | |
| 3.32 | 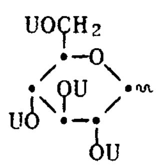 mit U = benzoyl<br>2,3,4,6-Tetra-O-benzoyl-($C_6$-D-glucose | |
| 3.33 | 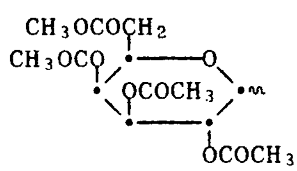<br>2,3,4,5-Tetra-O-acetyl-($C_6$-D-galactose) | $C_2H_5$ |

Tabelle 3 (Fortsetzung)

| Verb. Nr. | $R_1$ |
|---|---|
| 3.34 | 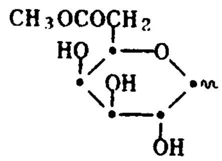 6-O-Acetyl-($C_6$-D-galactose) |
| 3.35 | 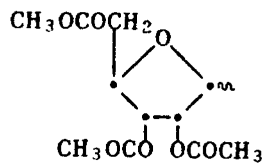 2,3,5-Tri-O-acetyl-($C_5$-D-ribose) |
| 3.36 | 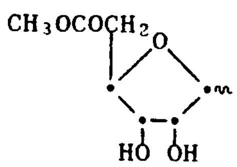 5-O-Acetyl-($C_5$-D-ribose) |
| 3.37 | 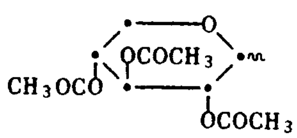 2,3,4-Tri-O-acetyl-($C_5$-D-xylose) |
| 3.38 | 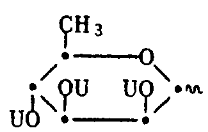 mit U=acetyl 2,3,4-Tri-O-acetyl-($C_6$-D-rhamnose) |

Formulierungsbeispiele für den Wirkstoff der Formel I

(% = Gewichtsprozent)

| Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man

erhält Spritzpulver, die sich mit Wasser zu Suspensionen jeder gewünschten Konzentration verdünnen lassen.

| Emulsions-Konzentrat | |
|---|---|
| Wirkstoff aus den Tabellen | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Extruder Granulat | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Tabellen bzw. Boli

| | | |
|---|---|---|
| I | Ein Wirkstoff aus den Tabellen | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Meisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen
Phasen I und II mischen und zu Tabletten oder Boli verpressen.
Falls die Verbindungen der Formel I bzw. entsprechende Mittel zur Bekämpfung von endoparasitären

Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tranken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24 °C und 60 % relativer Luftfeuchtigkeit durchgeführt. Auswertungen und Zwischenauswertungen auf moribunde Tiere, Wachstum und Larven und Frassschäden erfolgen nach 24 Stunden, 48 Stunden und 72 Stunden.

Verbindungen der Formeln I aus den Tabellen erzielten bereits bei einer Wirkstoffkonzentration von 3 ppm eine vollständige Abtötung nach 24 Stunden, so z.B. Nr. 1.14, 1.11, 1.22 und 1.23.

### B-2. Wirkung gegen pflanzenschädigende Akariden OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25 °C.

Nach sieben Tagen wird unter dem Binokular auf Prozentsatz mobiler Stadien (Adulte und Nymphen) und auf vorhandene Eier ausgewertet.

Verbindungen der Formel I aus den Tabellen, wie z.B. die Verbindungen Nr. 1.22 und 1.23 erzielten bereits bei einer Wirkstoffkonzentration von 0,4 ppm vollständige Abtötung.

### B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50 °C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen der Formel I aus den Tabellen, wie z.B. die Verbindungen Nr. 1.14, 1.15, 1.17, 1.22 und 1.23 erzielten mit 100 ppm eine Wirkung von 100 %.

### B-4. Akarizide Wirkung gegen Boophilus microplus (Biarra-Stamm

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 1, 0,1 oder 0,01 µg pro Zecke gelöst ist. Kontrolltiere erhalten eine wirkstofffreie Injektion. Nach der Behandlung werden die Tiere unter Normalbedingungen in einem Insektarium bei ca. 28 °C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formeln I aus den Tabellen, wie z.B. die Verbindungen Nr. 1.14, 1.15, 1.22 und 1.23 erzielen eine $IR_{90}$ von 0,1 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 0,5 mg oder 0,2 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formeln I wie z.B. mit der Nr. 1.14, 1.15, 1.17, 1.22, 1.23 oder 3.30 bei 0,2 mg/kg behandelt wurden, zeigten im Vergleich zu unbehandelten, aber infizierten Vergleichsgruppen keinen Nematodenbefall (= komplette Reduktion der Wurmeier im Kot).

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Verbindungen der Formel I aus den Tabellen, wie z.B. die Verbindungen Nr. 1.14, 1.15, 1.17, 1.22, 1.23 und 3,30 erzielten bei einer Konzentration von 12,5 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formeln I aus den Tabellen, wie z.B. die Verbindungen Nr. 1.14, 1.15, 1.17, 1.22, 1.23 und 3.30 bewirkten in diesem Test bei einer Konzentration von 1,6 ppm bereits nach einem Tag eine vollständige Abtötung sämtlicher Larven.

## Patentansprüche

**1.** Verbindungen der Formel I

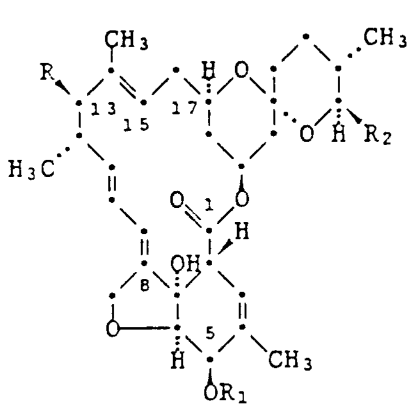

(I)

worin R für $C_1$-$C_{10}$-Alkyl steht; $R_1$ Wasserstoff, eine Silylgruppe oder den Zuckerrest

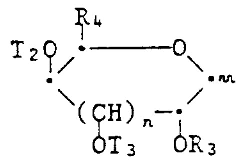

unter Einschluss seiner Stellungsisomeren repräsentiert, wobei n für die Zahl 0 oder 1 steht; $R_4$ Wasserstoff, Methyl oder -$CH_2$-O-$T_1$ bedeutet und $R_4$, $T_1$, $T_2$ und $T_3$ unabhängig voneinander Wasserstoff, Methyl, Benzyl, eine unsubstituierte oder halogensubstituierte $C_1$-$C_6$-aliphatische Acylgruppe, eine Benzoylgruppe, oder eine $C_1$-$C_6$-Alkoxycarbonylgruppe bedeuten, oder wobei $T_1$ und $T_2$ zusammen mit dem Carbonyl-Kohlenstoffatom eines aliphatischen oder aromatischen Aldehyds oder Ketons mit maximal 13 Kohlenstoffatomen ein cyclisches Acetal bilden, $R_3$ für Methyl, Benzyl, Benzoyl, unsubstituiertes oder durch Fluor substituiertes Propionyl, Acetyl, Methoxycarbonyl oder Ethoxicarbonyl steht und $R_2$ für Methyl, Ethyl, Isopropyl oder sek.-Butyl steht.

2. Verbindungen der Formel I nach Anspruch 1, worin R für $C_1$-$C_{10}$-Alkyl steht; $R_1$ die Gruppe -Si($R_5$)($R_6$)-($R_7$) repräsentiert, wobei $R_5$, $R_6$ und $R_7$ unabhängig voneinander für $C_1$-$C_4$-Alkyl, Benzyl oder Phenyl stehen; und $R_2$ für Methyl, Ethyl, Isopropyl oder sek.-Butyl steht.

3. Verbindungen der Formel I nach Anspruch 2 worin R für $C_1$-$C_4$-Alkyl steht; $R_1$ Trimethylsilyl, tris(tert.-Butyl)silyl, Diphenyl-tert.-butylsilyl, bis(Isopropyl)methylsilyl, Triphenylsilyl oder tert.-Butyl-dimethylsilyl bedeutet; und $R_2$ für Methyl, Ethyl, Isopropyl oder sek.-Butyl steht.

4. Verbindungen der Formel I nach Anspruch 1, worin $R_1$ für $C_1$-$C_4$-Alkyl steht; $R_3$ für Methyl, Benzyl, Benzoyl, unsubstituiertes oder durch Fluor substituiertes Propionyl, Acetyl, Methoxycarbonyl oder Ethoxicarbonyl bedeutet; und
$R_2$, $R_4$, $T_2$ und $T_3$ die in Anspruch 4 angegebenen Bedeutungen haben.

5. Verbindungen der Formel I nach Anspruch 1, worin R für $C_1$-$C_{10}$-Alkyl steht; $R_1$ Wasserstoff bedeutet; und $R_2$ für Methyl, Ethyl, Isopropyl oder sek.-Butyl steht.

6. Verbindungen der Formel I nach Anspruch 5, worin R für $C_1$-$C_6$-Alkyl steht; $R_1$ Wasserstoff bedeutet; und $R_2$ für Methyl, Ethyl, Isopropyl oder sek.-Butyl steht.

7. Verbindungen der Formel I nach Anspruch 6, worin R für $C_1$-$C_4$-Alkyl steht; $R_1$ Wasserstoff bedeutet; und $R_2$ für Methyl, Ethyl, Isopropyl oder sek.-Butyl steht.

8. Verbindungen der Formel I nach Anspruch 7, worin R für Methoxy, Ethyl, n-Propyl oder Isopropyl steht; $R_1$ Wasserstoff bedeutet und $R_2$ für Methyl, Ethyl oder Isopropyl steht.

9. Eine Verbindung der Formel I nach Anspruch 1 ausgewählt aus der Reihe:

13$\beta$-n-Hexyl-milbemycin D
13$\beta$-Methyl-milbemycin D,
13$\beta$-Ethyl-milbemycin D,
13$\beta$-n-Propyl-milbemycin $A_4$,
13$\beta$-iso-Propyl-milbemycin $A_4$,
13$\beta$-Methyl-milbemycin $A_3$,
13$\beta$-Ethyl-milbemycin $A_3$,
13$\beta$-Methyl-milbemycin $A_4$,
13$\beta$-Ethyl-milbemycin $A_4$,
13$\beta$-iso-Butyl-milbemycin $A_4$ und
13$\beta$-n-Butyl-milbemycin $A_4$.

**10.** Verfahren zur Herstellung von Verbindungen der Formel I nach Anspruch 1, dadurch gekennzeichnet, dass man einen Allylester der Formel II

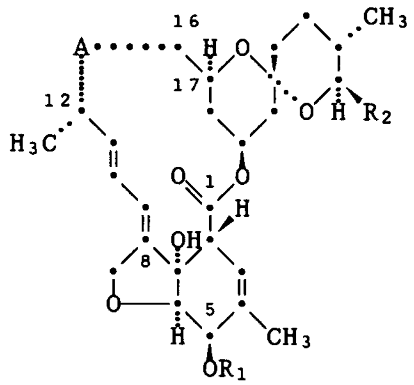

(II)

worin A für eine der Gruppen a oder b

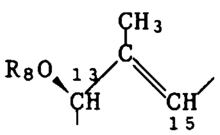 (a) oder 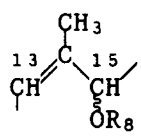 (b)

[= 13β-Ester-$\Delta^{14,15}$] [= $\Delta^{13,14}$-15-Ester]

steht, $R_8$ eine Acylgruppe repräsentiert, $R_1$ Wasserstoff oder eine Silylgruppe bedeutet und $R_2$ die unter Formel I angegebene Bedeutung hat, mit einer Trialkylaluminium-Verbindung der Formel III

$Al(R)_3$ (III)

behandelt, wobei R die unter Formel I angegebenen Bedeutungen hat, woraufhin man die $R_1$-Silylgruppe, sofern freie 5-Hydroxi-Verbindungen erwünscht sind, hydrolytisch abspaltet und zur Einführung des Zuckerrestes $R_1$ eine Hydroxi-Verbindung der Formel I mit einem zur Einführung dieser Gruppe geeigneten Zuckerderivat umsetzt.

**11.** Verfahren nach Anspruch 10, dadurch gekennzeichnet, dass man die Umsetzung von II und III in einem reaktionsinerten Lösungsmittel bei Temperaturen von -100 °C bis +100 °C, vorzugsweise -20 °C bis +60 °C durchführt.

**12.** Schädlingebekämpfungsmittel gegen Ekto-, Endoparasiten und Insekten, dass es neben üblichen Trägerstoffen und/oder Verteilungsmitteln mindestens eine Verbindung der Formel I nach Anspruch 1 enthält.

**13.** Mittel gemäss Anspruch 12, dadurch gekennzeichnet, dass es als Verbindung der Formel I mindestens eine Verbindung gemäss den Ansprüchen 2 bis 9 enthält.

**14.** Verfahren zur Bekämpfung von Schädlingen an Pflanzen, dadurch gekennzeichnet, dass man eine Verbindung der Formel I nach Anspruch 1, auf die Pflanze oder deren Lebensraum appliziert.

**15.** Verfahren nach Anspruch 14, dadurch gekennzeichnet, dass es sich bei der applizierten Substanz um eine Verbindung der Formel I gemäss einem der Ansprüche 2 bis 9 handelt.

**16.** Verfahren nach Anspruch 15, dadurch gekennzeichnet, dass es sich um phytopathogene Ektoparasiten und Insekten handelt.

17. Verbindungen der Formel I nach Anspruch 1 zur Verwendung in einem Verfahren zur Bekämpfung von Ekto-, Endoparasiten und Insekten am Nutztier.

18. Verbindungen der Formel I nach Anspruch 1 zur Verwendung bei der Herstellung von veterinärmedizinischen Präparaten zur Bekämpfung von Ekto-, Endoparasiten und Insekten am Nutztier.

## Claims

1. A compound of formula I

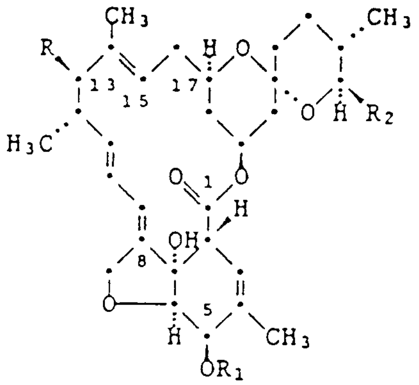

(I)

wherein R is $C_1$-$C_{10}$alkyl; $R_1$ is hydrogen, a silyl group or the sugar residue

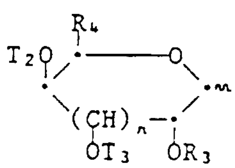

including the position isomers thereof, in which n is the number 0 or 1; $R_4$ is hydrogen, methyl or -$CH_2$-O-$T_1$, and $R_4$, $T_1$, $T_2$ and $T_3$ independently of one another are hydrogen, methyl, benzyl, an unsubstituted or halo-substituted aliphatic $C_1$-$C_6$acyl group, a benzoyl group, or a $C_1$-$C_6$alkoxycarbonyl group, or in which $T_1$ and $T_2$ together with the carbon atom of the carbonyl group of an aliphatic or aromatic aldehyde or ketone form a cyclic acetal containing not more than 13 carbon atoms, $R_3$ is methyl, benzyl, benzoyl, unsubstituted or fluoro-substituted propionyl, acetyl, methoxycarbonyl or ethoxycarbonyl and $R_2$ is methyl, ethyl, isopropyl or sec-butyl.

2. A compound of formula I according to claim 1, wherein R is $C_1$-$C_{10}$alkyl; $R_1$ is the group -Si($R_5$)($R_6$)-($R_7$), in which $R_5$, $R_6$ and $R_7$ independently of one another are $C_1$-$C_4$alkyl, benzyl or phenyl; and $R_2$ is methyl, ethyl, isopropyl or sec-butyl.

3. A compound of formula I according to claim 2, wherein R is $C_1$-$C_4$-alkyl; $R_1$ is trimethylsilyl, tris(tert-butyl)silyl, diphenyl-tert-butylsilyl, bis(isopropyl)methylsilyl, triphenylsilyl or tert-butyldimethylsilyl; and $R_2$ is methyl, ethyl, isopropyl or sec-butyl.

4. A compound of formula I according to claim 1, wherein $R_1$ is $C_1$-$C_4$alkyl; $R_3$ is methyl, benzyl, benzoyl, unsubstituted or fluoro-substituted propionyl, acetyl, methoxycarbonyl or ethoxycarbonyl; and $R_2$, $R_4$, $T_2$ and $T_3$ are as defined in claim 4.

5. A compound of formula I according to claim 1, wherein R is $C_1$-$C_{10}$alkyl; $R_1$ is hydrogen; and $R_2$ is methyl, ethyl, isopropyl or sec-butyl.

**6.** A compound of formula I according to claim 5, wherein R is $C_1$-$C_6$alkyl; $R_1$ is hydrogen; and $R_2$ is methyl, ethyl, isopropyl or sec-butyl.

**7.** A compound of formula I according to claim 6, wherein R is $C_1$-$C_4$alkyl; $R_1$ is hydrogen; and $R_2$ is methyl, ethyl, isopropyl or sec-butyl.

**8.** A compound of formula I according to claim 7, wherein R is methyl, ethyl, n-propyl or isopropyl; $R_1$ is hydrogen and $R_2$ is methyl, ethyl or isopropyl.

**9.** A compound of formula I according to claim 1, selected from the series consisting of:

13β-n-hexylmilbemycin D,
13β-methylmilbemycin D,
13β-ethylmilbemycin D,
13β-n-propylmilbemycin $A_4$,
13β-isopropylmilbemycin $A_4$,
13β-methylmilbemycin $A_3$,
13β-ethylmilbemycin $A_3$,
13β-methylmilbemycin $A_4$,
13β-ethylmilbemycin $A_4$,
13β-isobutylmilbemycin $A_4$ and
13β-n-butylmilbemycin $A_4$.

**10.** A process for the preparation of a compound of formula I according to claim 1, which comprises treating an allyl ester of formula II

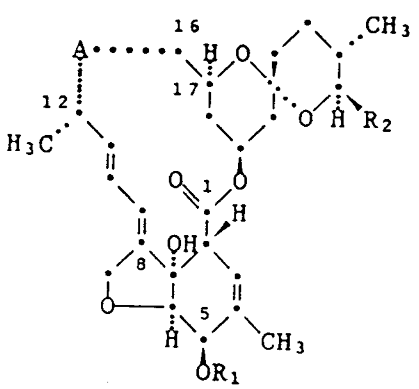

(II)

wherein A is one of the groups a or b

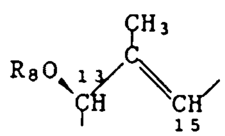 (a) or 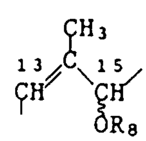 (b)

[= 13β-ester-$\Delta^{14,15}$] [= $\Delta^{13,14}$-15-ester],

$R_8$ is an acyl group, $R_1$ is hydrogen or a silyl group and $R_2$ is as defined for formula I, with a trialkylaluminium compound of formula III

$Al(R)_3$ (III)

in which R is as defined for formula I, and, if a free 5-hydroxy compound is desired, subsequently

removing the silyl group $R_1$ by hydrolysis and, to introduce the sugar residue $R_1$, reacting a hydroxy compound of formula I with a sugar derivative suitable for introducing this group.

**11.** A process according to claim 10, which comprises carrying out the reaction of II and III in a solvent which is inert in the reaction at temperatures from -100°C to +100°C, preferably from -20°C to +60°C.

**12.** A pesticidal composition for controlling ectoparasites, endoparasites and insects, which contains at least one compound of formula I according to claim 1 in addition to customary carriers and/or dispersing agents.

**13.** A composition according to claim 12, which contains as compound of formula I at least one compound according to any one of claims 2 to 9.

**14.** A method of controlling pests on plants, which comprises applying to the plant or its habitat a compound of formula I according to claim 1.

**15.** A method according to claim 14, wherein the substance applied is a compound of formula I according to any one of claims 2 to 9.

**16.** A method according to claim 15, relating to phytopathogenic ectoparasites and insects.

**17.** A compound of formula I according to claim 1 for use in a method of controlling ectoparasites, endoparasites and insects on productive livestock.

**18.** A compound of formula I according to claim 1 for use in the production of veterinary preparations for controlling ectoparasites, endoparasites and insects on productive livestock.

**Revendications**

**1.** Composés de formule I

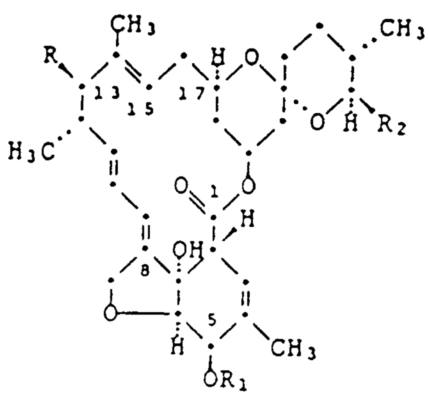

(I)

dans laquelle R représente un alkyle en $C_{1-10}$, $R_1$ un hydrogène, un groupe silyle ou le radical sucré

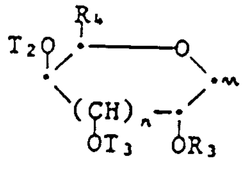

y compris ses isomères de position, où n représente le nombre 0 ou 1, $R_4$ représente un hydrogène, un méthyle ou-$CH_2$-O-$T_1$ et $R_4$, $T_1$, $T_2$ et $T_3$ représentent indépendamment l'un de l'autre un

hydrogène, méthyle, benzyle, un groupe acyle aliphatique en $C_{1-6}$ non substitué ou halogéno-substitué, un groupe benzoyle, ou un groupe alcoxycarbonyle en $C_{1-6}$, ou bien où $T_1$ et $T_2$ forment ensemble avec l'atome de carbone d'un carbonyle d'un aldéhyde ou d'une cétone aliphatique ou aromatique ayant au maximum 13 atomes de carbone, un acétal cyclique, $R_3$ représente un méthyle, benzyle, benzoyle, propionyle non substitué ou substitué par un fluor, acétyle, méthoxycarbonyle ou éthoxycarbonyle et $R_2$ représente un méthyle, éthyle, isopropyle, ou sec.-butyle.

**2.** Composés de formule I selon la revendication 1, où R représente un alkyle en $C_{1-10}$ ; $R_1$ représente le groupe -Si($R_5$)($R_6$)($R_7$), où $R_5$, $R_6$ et $R_7$ représentent indépendamment l'un de l'autre un alkyle en $C_{1-4}$, un benzyle ou un phényle ; et $R_2$ représente un méthyle, éthyle, isopropyle ou sec.-butyle.

**3.** Composés de formule I selon la revendication 2 où R représente un alkyle en $C_{1-4}$ ; $R_1$ représente un triméthylsilyle, tris(tert.-butyl)silyle, diphényl-tert.-butyl-silyle, bis(isopropyl)méthylsilyle, triphénylsilyle ou tert.-butyl-di-méthylsilyle ; et $R_2$ un méthyle, éthyle, isopropyle ou sec.-butyle.

**4.** Composés de formule I selon la revendication 1 où $R_1$ représente un alkyle en $C_{1-4}$ ; $R_3$ représente un méthyle, benzyle, benzoyle, propionyle non substitué ou substitué par un fluor, acétyle, méthoxycarbonyle ou éthoxycarbonyle ; et $R_2$, $R_4$, $T_2$ et $T_3$ ont les significations données dans la revendication 4.

**5.** Composés de formule I selon la revendication 1, où R représente un alkyle en $C_{1-10}$, $R_1$ représente un hydrogène ; et $R_2$ représente un méthyle, éthyle, isopropyle ou sec.-butyle.

**6.** Composés de formule I selon la revendication 5, où R représente un alkyle en $C_{1-6}$ ; $R_1$ représente un hydrogène ; et $R_2$ représente un méthyle, éthyle, isopropyle ou sec.-butyle.

**7.** Composés de formule I selon la revendication 6, où R représente un alkyle en $C_{1-4}$ ; $R_1$ représente un hydrogène ; et $R_2$ représente un méthyle, éthyle, isopropyle ou sec.-butyle.

**8.** Composés de formule I selon la revendication 7, où R représente un méthoxy, éthyle, n-propyle ou isopropyle ; $R_1$ représente un hydrogène et $R_2$ représente un méthyle, éthyle ou isopropyle.

**9.** Composé de formule I selon la revendication 1 choisi dans la série :
13$\beta$-n-hexyl-milbémycine D
13$\beta$-méthyl-milbémycine D,
13$\beta$-éthyl-milbémycine D,
13$\beta$-n-propyl-milbémycine $A_4$,
13$\beta$-iso-propyl-milbémycine $A_4$,
13$\beta$-méthyl-milbémycine $A_3$,
13$\beta$-éthyl-milbémycine $A_3$,
13$\beta$-méthyl-milbémycine $A_4$,
13$\beta$-éthyl-milbémycine $A_4$,
13$\beta$-iso-butyl-milbémycine $A_4$, et
13$\beta$-n-butyl-milbémycine $A_4$.

**10.** Procédé de préparation de composés de formule I selon la revendication 1, caractérisé en ce qu'on traite un allylester de formule II

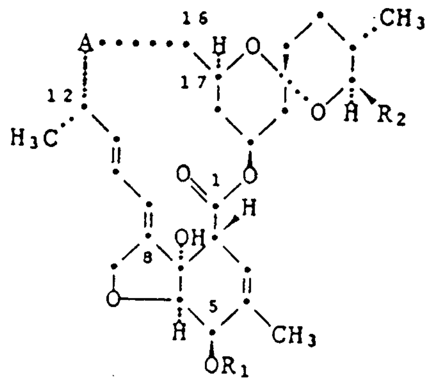

(II)

où A représente l'un des groupes a ou b

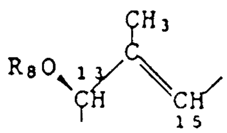 (a) ou 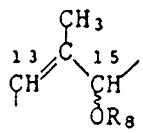 (b)

[= 13β-Ester-$\Delta^{14,15}$] [= $\Delta^{13,14}$-15-Ester]

$R_8$ représente un groupe acyle, $R_1$ représente un hydrogène ou un groupe silyle et $R_2$ a la signification donnée sous la formule I, avec un composé de trialkylaluminium de formule III

$Al(R_3)$ (III)

où R a les significations données sous la formule I, après quoi on sépare par hydrolyse le groupe silyle $R_1$ dans la mesure où l'on désire des composés 5-hydroxy, et pour l'introduction du radical sucré $R_1$ on fait réagir un composé hydroxy de formule I avec un dérivé sucré approprié à l'introduction de ce groupe.

**11.** Procédé selon la revendication 10, caractérisé en ce qu'on conduit la réaction de II et de III dans un solvant inerte vis-à-vis de la réaction à des températures allant de -100 ° C à +100 ° C, de préférence de -20 ° C à +60 ° C.

**12.** Agent de lutte antiparasitaire contre les ecto-, endoparasites et les insectes, caractérisé en ce qu'il contient outre les supports ou agents de répartition habituels au moins un composé de formule I selon la revendication 1.

**13.** Agent selon la revendication 12, caractérisé en ce qu'il contient comme composé de formule I au moins un composé selon les revendications 2 à 9.

**14.** Procédé de lutte contre les parasites des plantes, caractérisé en ce qu'on applique un composé de formule I selon la revendication 1 aux plantes ou à leur habitat.

**15.** Procédé selon la revendication 14, caractérisé en ce qu'il s'agit, pour la substance appliquée, d'un composé de formule I selon l'une des revendications 2 à 9.

**16.** Procédé selon la revendication 15, caractérisé en ce qu'il s'agit, pour les agents phytopathogènes, d'ectoparasites et d'insectes.

**17.** Composés de formule I selon la revendication 1, aux fins d'utilisation dans un procédé de lutte contre les ecto-, endoparasites et les insectes chez les animaux utiles.

**18.** Composés de formule I selon la revendication 1 aux fins d'utilisation dans la préparation de préparations de médecine vétérinaire pour combattre les ecto-, endoparasites et les insectes chez les animaux utiles.